(19) Europäisches Patentamt
European Patent Office
Office européen des brevets

(11) EP 0 658 562 B1

(12) EUROPÄISCHE PATENTSCHRIFT

(45) Veröffentlichungstag und Bekanntmachung des Hinweises auf die Patenterteilung:
**10.11.1999 Patentblatt 1999/45**

(51) Int Cl.[6]: **C07F 9/24**, A01N 57/30, C07C 237/04

(21) Anmeldenummer: **94118927.6**

(22) Anmeldetag: **01.12.1994**

# (54) Phosphorsäure-Derivate als Schädlingsbekämpfungsmittel

Pesticidal phosphoric acid derivatives

Dérivés d'acide phosphorique pesticides

(84) Benannte Vertragsstaaten:
**BE CH DE ES FR GB IT LI NL**

(30) Priorität: **14.12.1993 DE 4342621**

(43) Veröffentlichungstag der Anmeldung:
**21.06.1995 Patentblatt 1995/25**

(73) Patentinhaber: **BAYER AG**
**51368 Leverkusen (DE)**

(72) Erfinder:
- **Maurer, Fritz, Dr.**
  **Oyama-shi, Tochigi 323 (JP)**
- **Erdelen, Christoph, Dr.**
  **D-42799 Leichlingen (DE)**
- **Wachendorff-Neumann, Ulrike Dr.**
  **D-53177 Bonn (DE)**
- **Hartwig, Jürgen, Dr.**
  **D-42799 Leichlingen (DE)**
- **Turberg, Andreas, Dr.**
  **D-40699 Erkrath (DE)**
- **Mencke, Norbert, Dr.**
  **D-51381 Leverkusen (DE)**

(56) Entgegenhaltungen:
**GB-A- 2 164 940** **US-A- 4 889 944**

- **CHEMICAL ABSTRACTS, vol. 093, no. 23, 8. Dezember 1980, Columbus, Ohio, US; abstract no. 220697, YANG X Z ET AL 'Studies on drugs for coronary heart disease. I. Synthesis of ring compounds with substituted aminoacetylamino group' & YAO HSUEH HSUEH PAO (YHHPAL,05134870);80; VOL.15 (1); PP.18-26, ACAD. SIN.;SHANGHAI INST. MATER. MED.; SHANGHAI; PEOP. R. CHINA**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 658 562 B1

Printed by Jouve, 75001 PARIS (FR)

## Beschreibung

Die Erfindung betrifft neue Phosphorsäure-Derivate, ein Verfahren zu ihrer Herstellung sowie ihre Verwendung als Schädlingsbekämpfungsmittel, insbesondere als Insektizide, Akarizide und Nematizide.

Es ist bekannt, daß bestimmte O,O-disubstituierte Thiophosphoryl-N,N-glycinamide insektizide, akarizide und nematizide Eigenschaften aufweisen (vgl. US-A-4 870 065 und US-A-4 889 944). Die Wirksamkeit dieser bekannten Verbindungen ist jedoch unter bestimmten Umständen, insbesondere bei niedrigen Wirkstoffkonzentrationen und Aufwandmengen, nicht immer ganz zufriedenstellend.

Es wurden nun die neuen Phosphorsäure-Derivate der allgemeinen Formel (I)

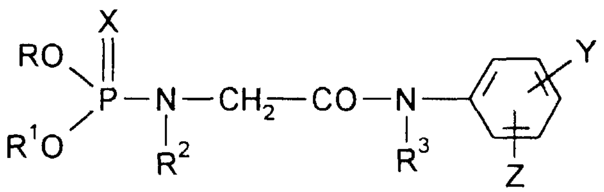

(I)

gefunden, in welcher

| | |
|---|---|
| R | für gegebenenfalls substituiertes Alkyl steht; |
| $R^1$ | für gegebenenfalls substituiertesAlkyl steht; |
| $R^2$ | für gegebenenfalls substituiertes Alkyl oder gegebenenfalls substituiertes Alkenyl steht; |
| $R^3$ | für gegebenenfalls substituiertes $C_4$-$C_6$-Alkyl steht; |
| X | für Sauerstoff oder Schwefel steht, und |
| Y und Z | für gleiche oder verschiedene Reste aus der Reihe Wasserstoff, Halogen, Alkyl und Alkoxy stehen. |

Weiter wurde gefunden, daß man die neuen Phosphorsäure-Derivate der allgemeinen Formel (I) erhält, wenn man

a) Verbindungen der allgemeinen Formel (II)

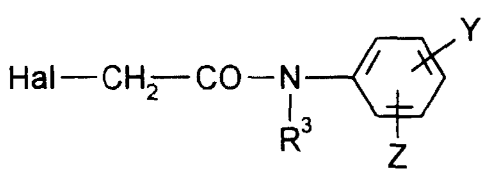

(II)

in welcher

| | |
|---|---|
| $R^3$, | Y und X die oben angegebenen Bedeutungen haben, |
| Hal | für Halogen (vorzugsweise Chlor) steht, |

mit Verbindungen der Formel (III)

$$R^2\text{-}NH_2 \qquad \text{(III)}$$

in welcher

$R^2$ die oben angegebene Bedeutung hat,

umsetzt, und

b) die im Reaktionsschritt a) erhaltenen Verbindungen der allgemeinen Formel (IV)

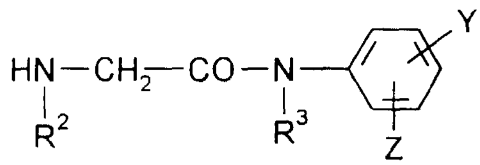

(IV)

in welcher

$R^2$, $R^3$, Y und Z die oben angegebenen Bedeutungen haben,

gegebenenfalls nach ihrer Isolierung mit Verbindungen der allgemeinen Formel (V)

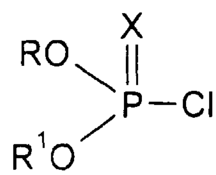

(V)

in welcher

R, $R^1$ und X die oben angegebenen Bedeutungen haben,

gegebenenfalls in Gegenwart eines Säureakzeptors und gegebenenfalls in Gegenwart eines Verdünnungsmittels umsetzt.

Die neuen Verbindungen der allgemeinen Formel (I) verfügen über Eigenschaften, die ihre Verwendung als Schädlingsbekämpfungsmittel ermöglichen. Sie zeigen starke Wirkung gegen Arthropoden und Nematoden und können insbesondere zur Bekämpfung von Insekten, Milben und Nematoden verwendet werden.

Bevorzugte Substituenten bzw. Bereiche der in den oben und nachstehend erwähnten Formeln aufgeführten Reste werden im folgenden erläutert:

In den allgemeinen Formeln bedeutet Alkyl als solches oder der Bestandteil eines anderen Restes, z.B. Alkoxy, geradkettiges oder verzweigtes Alkyl mit vorzugsweise 1 bis 8 Kohlenstoffatomen, insbesondere mit 1 bis 6 Kohlenstoffatomen, besonders bevorzugt mit 1 bis 5 und ganz besonders bevorzugt mit 1 bis 4 Kohlenstoffatomen. Als bevorzugt seien genannt: Methyl, Ethyl, n- und i-Propyl, n-, i-, s- und t-Butyl, Pentyl, Hexyl und Octyl. Der Alkylrest $R^3$ ist geradkettig oder verzweigt (vorzugsweise verzweigt) und enthält vorzugsweise 4 oder 5 (vorzugsweise 4) Kohlenstoffatome, wobei bevorzugt genannt seien n.-, i.-, s.- und t-Butyl und besonders bevorzugt s.- und i.-Butyl.

In den allgemeinen Formeln bedeutet Alkenyl geradkettiges oder verzeigtes Alkenyl mit 2 bis 6, insbesondere 2 bis 4 Kohlenstoffatomen, wobei als besonders bevorzugt der Allylrest genannt sei.

In den allgemeinen Formeln bedeutet Halogen, Y und Z Fluor, Chlor, Brom und/oder Iod, vorzugsweise Fluor, Chlor und/oder Brom und besonders bevorzugt Fluor und/oder Chlor. Besonders bevorzugt steht einer der Reste X und Z für Wasserstoff und ganz besonders bevorzugt stehen beide Y und Z für Wasserstoff.

Die Alkylreste R, $R^1$, $R^2$, $R^3$ sowie der Alkenylrest $R^2$ können ein- oder mehrfach (vorzugsweise ein- bis 5-fach, insbesondere ein- bis 3-fach) durch gleiche oder verschiedene Substituenten substituiert sein, wobei als bevorzugte Substituenten Halogen (vorzugsweise Fluor, Chlor, Brom und/oder Iod, insbesondere Fluor und/oder Chlor) und/oder Alkoxy mit vorzugsweise 1 bis 4, insbesondere 1 oder 2 Kohlenstoffatomen genannt seien. Besonders bevorzugt sind die Alkylreste R, $R^1$, $R^2$ und $R^3$ sowie der Alkenylrest $R^2$ unsubstituiert. In einer weiteren bevorzugten Ausführungsform trägt der Alkylrest $R^2$ einen Alkoxyrest mit vorzugsweise 1 oder 2 Kohlenstoffatomen.

In den allgemeinen Formeln steht X vorzugsweise für Schwefel.

In den allgemeinen Formeln steht

R vorzugsweise für $C_1$-$C_6$-Alkyl, welches gegebenenfalls durch Halogen (vorzugsweise Fluor und/oder Chlor) und/oder $C_1$-$C_4$-Alkoxy substituiert ist.

In den allgemeinen Formeln steht

$R^1$ vorzugsweise für $C_1$-$C_6$-Alkyl, welches gegebenenfalls durch Halogen (vorzugsweise Fluor und/oder Chlor) und/oder $C_1$-$C_4$-Alkoxy substituiert ist.

In den allgemeinen Formeln steht

$R^2$ vorzugsweise für $C_1$-$C_6$-Alkyl oder $C_2$-$C_6$-Alkenyl, wobei diese Reste gegebenenfalls durch Halogen (vorzugsweise Fluor und/oder Chlor) und/oder $C_1$-$C_4$-Alkoxy substituiert sind.

In den allgemeinen Formeln steht

$R^3$ vorzugsweise für $C_4$-$C_6$-Alkyl, welches gegebenenfalls durch Halogen (vorzugsweise Fluor und/oder Chlor) und/oder $C_1$-$C_4$-Alkoxy substituiert ist.

In den allgemeinen Formeln stehen

Y und Z vorzugsweise für Wasserstoff, Halogen (vorzugsweise Fluor und/oder Chlor) und/oder für gleiche oder verschiedene Reste aus der Reihe $C_1$-$C_6$-Alkyl und $C_1$-$C_6$-Alkoxy.

In den allgemeinen Formeln steht

R besonders bevorzugt für $C_1$-$C_4$-Alkyl, welches gegebenenfalls durch Halogen (vorzugsweise Fluor und/oder Chlor) und/oder $C_1$-$C_4$-Alkoxy substituiert ist.

In den allgemeinen Formeln steht

$R^1$ besonders bevorzugt für $C_1$-$C_4$-Alkyl, welches gegebenenfalls durch Halogen (vorzugsweise Fluor und/oder Chlor) und/oder $C_1$-$C_4$-Alkoxy substituiert ist:

In den allgemeinen Formeln steht

$R^2$ besonders bevorzugt für $C_1$-$C_4$-Alkyl oder $C_2$-$C_4$-Alkenyl, wobei diese Reste gegebenenfalls durch Halogen (vorzugsweise Fluor und/oder Chlor) und/oder $C_1$-$C_4$-Alkoxy substituiert sind.

In den allgemeinen Formeln steht

$R^3$ besonders bevorzugt für $C_4$-$C_5$-Alkyl, welches gegebenenfalls durch Halogen (vorzugsweise Fluor und/oder Chlor) und/oder $C_1$-$C_4$-Alkoxy substituiert ist.

In den allgemeinen Formeln stehen

Y und Z besonders bevorzugt für Wasserstoff, Halogen (vorzugsweise Fluor und/oder Chlor), und/oder für gleiche oder verschiedene Reste aus der Reihe $C_1$-$C_4$-Alkyl und $C_1$-$C_4$-Alkoxy.

In den allgemeinen Formeln steht

R ganz besonders bevorzugt für $C_1$-$C_4$-Alkyl, welches gegebenenfalls durch Halogen (vorzugsweise Fluor und/oder Chlor) und/oder $C_1$-$C_2$-Alkoxy substituiert ist.

In den allgemeinen Formeln steht

$R^1$ ganz besonders bevorzugt für $C_1$-$C_4$-Alkyl, welches gegebenenfalls durch Halogen (vorzugsweise Fluor und/oder

Chlor) und/oder $C_1$-$C_2$-Alkoxy substituiert.

In den allgemeinen Formeln steht

$R^2$ ganz besonders bevorzugt für $C_1$-$C_4$-Alkyl oder $C_2$-$C_4$-Alkenyl, wobei diese Reste gegebenenfalls durch Halogen (vorzugsweise Fluor und/oder Chlor) und/oder $C_1$-$C_2$-Alkoxy substituiert sind.

In den allgemeinen Formeln steht

$R^3$ ganz besonders bevorzugt für $C_4$-$C_5$-Alkyl, welches gegebenenfalls durch Halogen (vorzugsweise Fluor und/oder Chlor) und/oder $C_1$-$C_2$-Alkoxy substituiert ist:

In den allgemeinen Formeln stehen

Y und Z ganz besonders bevorzugt für Wasserstoff, Halogen (vorzugsweise Fluor und/oder Chlor) und/oder für gleiche oder verschiedene Reste aus der Reihe $C_1$-$C_2$-Alkyl und $C_1$-$C_2$-Alkoxy.

In den allgemeinen Formeln steht

R besonders hervorgehoben für $C_1$-$C_4$-Alkyl.

In den allgemeinen Formeln steht

$R^1$ besonders hervorgehoben für $C_1$-$C_4$-Alkyl.

In den allgemeinen Formeln steht

$R^2$ besonders hervorgehoben für $C_1$-$C_4$-Alkyl, $C_1$-$C_2$-Alkoxy-$C_1$-$C_4$-Alkyl oder Allyl.

In den allgemeinen Formeln steht

$R^3$ besonders hervorgehoben für $C_4$-Alkyl.

In den allgemeinen Formeln stehen

Y und Z besonders hervorgehoben für Wasserstoff oder Y steht für Wasserstoff und Z steht für Fluor oder Chlor.

In den allgemeinen Formeln steht

R ganz besonders hervorgehoben für Methyl, Ethyl oder n-Propyl, insbesondere für Ethyl.

In den allgemeinen Formeln steht

$R^1$ ganz besonders hervorgehoben für Methyl, Ethyl, n-Propyl und iso-Propyl, insbesondere für Ethyl oder i-Propyl.

In den allgemeinen Formeln steht

$R^2$ ganz besonders hervorgehoben für Ethyl, i- und n-Propyl, n-, i- und s-Butyl, 2-Methoxyethyl oder Allyl, insbesondere für n-Propyl.

In den allgemeinen Formeln steht

$R^3$ ganz besonders bevorzugt hervorgehoben für n-, i-, s- oder t-Butyl, vorzugsweise für n-, i- oder s-Butyl und insbesondere für i- oder s-Butyl.

In den allgemeinen Formeln stehen

Y und Z ganz besonders bevorzugt für Wasserstoff.

In den allgemeinen Formeln steht

X ganz besonders bevorzugt hervorgehoben für Schwefel.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.
Erfindungsgemäß bevorzugt werden die Verbindungen der allgemeinen Formel (I), in welchen eine Kombination der vorstehend als bevorzugt (vorzugsweise) aufgeführten Bedeutungen vorliegt.
Erfindungsgemäß besonders bevorzugt werden die Verbindungen der allgemeinen Formel (I), in welchen eine Kombination der vorstehend als besonders bevorzugt aufgeführten Bedeutungen vorliegt.
Erfindungsgemäß ganz besonders bevorzugt werden die Verbindungen der allgemeinen Formel (I) verwendet, in welchen eine Kombination dieser vorstehend als ganz besonders bevorzugt aufgeführten Bedeutungen vorliegt.
Erfindungsgemäß besonders hervorgehoben werden die Verbindungen der allgemeinen Formel (I), in welchen eine Kombination der vorstehend als besonders hervorgehoben aufgeführten Bedeutungen vorliegt.
Erfindungsgemäß ganz besonders hervorgehoben werden die Verbindungen der allgemeinen Formel (I), in welcher eine Kombination der vorstehend als ganz besonders hervorgehoben aufgeführten Bedeutungen vorliegt.
Verwendet man beispielsweise Chloressigsäure-N-isobutylanilid (2) und N-Butylamin (3) als Ausgangsstoffe, so kann der Reaktionsablauf beim erfindungsgemäßen Verfahrensschritt (a) durch das folgende Formelschema wiedergegeben werden:

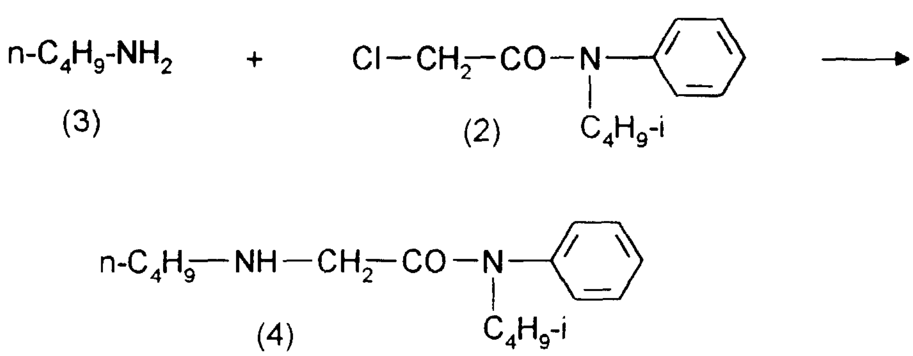
(3) (2)
(4)

Verwendet man beispielsweise das auf diese Weise erhaltene N,N-Butylaminoessigsäure-N-isobutylanilid (4) und Thiophosphorsäure-chlorid-O,O-diethylester (5) als Ausgangsstoffe, so kann der Reaktionsablauf bei der erfindungsgemaßen Verfahrensstufe (b) durch das folgende Formelschema wiedergegeben werden:

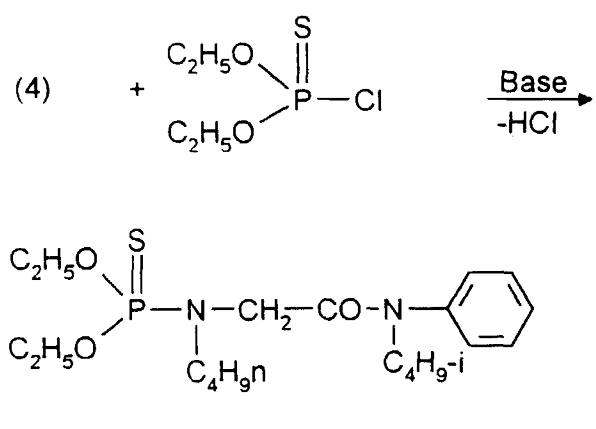

(4)

(1)

Die Ausgangsstoffe der allgemeinen Formeln II, III und V sind bekannt oder können nach bekannten Verfahren

und Methoden erhalten werden.

Die Ausgangsstoffe der allgemeinen Formel (IV) sind neu. Sie und das Verfahren zu ihrer Herstellung gemäß Verfahrensschritt a) sowie ihre Verwendung als Zwischenprodukte vorzugsweise zur Herstellung der Verbindungen der allgemeinen Formel (I) sind Teil der vorliegenden Erfindung.

Die erfindungsgemäßen Verfahrensstufen a) und b) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlond, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methylethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Als Säureakzeptoren können bei der erfindungsgemäßen Verfahrensstufe b) alle üblicherweise für derartige Umsetzungen verwendbaren Säurebindemittel eingesetzt werden. Vorzugsweise infrage kommen Alkalimetall- und Erdalkalimetallhydride, wie Lithium-, Natrium-, Kalium- und Calcium-hydrid, Alkalimetall- und Erdalkalimetall-hydroxide, wie Lithium-, Natrium-, Kalium- und Calciumhydroxid, Alkalimetall- und Erdalkalimetall-carbonate und -hydrogencarbonate, wie Natrium- und Kalium-carbonat oder -hydrogencarbonat sowie Calciumcarbonat, Alkalimetallacetate, wie Natrium- und Kalium-acetat, Alkalimetallalkoholate, wie Natrium- und Kalium-tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyl dicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methylpyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei den erfindungsgemäßen Verfahrensstufen a) und b) in einem größeren Bereich variiert werden. Im allgemeinen arbeitet man bei Temperaturen zwischen 0°C und 100°C, vorzugsweise bei Temperaturen zwischen 20°C und 80°C.

Die erfindungsgemäßen Verfahrensstufen werden im allgemeinen unter Normaldruck durchgeführt. Es ist jedoch auch möglich, unter erhöhtem oder vermindertem Druck zu arbeiten.

Zur Durchführung der erfindungsgemäßen Verfahrensstufen werden die jeweils benötigten Ausgangsstoffe bevorzugt in angenähert äquimolaren Mengen eingesetzt. Es ist jedoch auch möglich, eine der jeweils eingesetzten Komponenten in einem größeren Überschuß zu verwenden. Die Reaktionen werden in einem geeigneten Verdünnungsmittel und bei der Stufe b) in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahrensvarianten jeweils nach üblichen Methoden (vgl. die Herstellungsbeispiele).

Die Verbindungen der allgemeinen Formel (IV) werden vorzugsweise vor der weiteren Umsetzung gemäß Verfahrensstufe b) isoliert.

Die neuen Verbindungen der Formeln (I) und (IV) fallen in den meisten Fällen in Form von Ölen an, die sich zum Teil nicht unzersetzt destillieren lassen, jedoch durch sogenanntes "Andestillieren", d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung kann beispielsweise der Retentionsindex oder bei den Verbindungen der Formel (I) die Verschiebung im $^{31}$P-NMR-Spektrum dienen.

Die Wirkstoffe der Formel (I) eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis,

Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor,

Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxy-methylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von unerwünschten Schädlingen, wie Insekten, Zecken, Milben auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäßen Wirkstoffe geschieht auf diesen Gebieten in bekannter Weise, wie durch äußerliche Anwendung in Form beispielsweise des Tauchens (Dippen), Sprühens (Sprayen) und Aufgießens (pour-on and spot-on).

Die Herstellung der erfindungsgemäßen Verbindungen der Formel (I) soll anhand der folgenden Beispiele erläutert werden:

Prozentangaben beziehen sich, wo nichts anderes angegeben wird, auf Gewichtsprozente.

**Herstellungsbeispiele:**

**Beispiel 1**

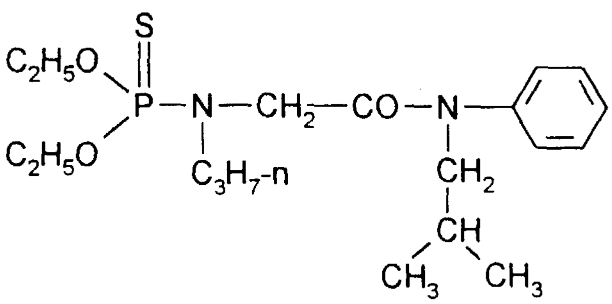

Zu einer Lösung von 6,2 g (0,025 Mol) N-n-Propylamino-essigsäure-N-isobutylanilid und 3,8 g (0,0375 Mol) Triethylamin in 50 ml wasserfreiem Methylenchlorid gibt man bei Raumtemperatur (ca. 20°C) 4,7 g (0,025 Mol) Thiophosphorsäure-chlorid-O,O-diethylester und rührt die Mischung 18 Stunden bei Raumtemperatur (ca. 20°C) nach. Dann extrahiert man das Reaktionsgemisch zweimal mit je 50 ml Wasser. Die organische Phase wird über Natriumsulfat getrocknet und im Vakuum vom Lösungsmittel befreit. Zurück bleiben 9,1 g (91 % der Theorie) N-n-Propyl-N-(O,O-diethyl-thiophosphoryl)-aminoessigsäure-N-isobutyl-anilid in Form eines Öles mit einer Verschiebung von 76,327 ppm im $^{31}P$-NMR.

Gemäß Beispiel 1 werden die folgenden Verbindungen der Formel (I)

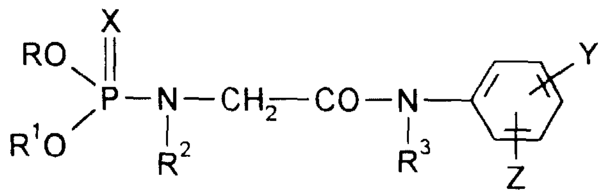

(I)

erhalten.

Tabelle 1

| Bsp.Nr. | R | $R^1$ | $R^2$ | $R^3$ | X | Y | Z | $^{31}P$-NMR Verschiebung (ppm) bzw. Brechungs-index $n_D^{20}$ |
|---|---|---|---|---|---|---|---|---|
| 2 | $C_2H_5$ | i-$C_3H_7$ | n-$C_3H_7$ | i-$C_4H_9$ | S | H | H | 74,913 |
| 3 | " | $C_2H_5$ | " | n-$C_4H_9$ | S | H | H | 76,358 |
| 4 | " | " | $C_2H_5$ | i-$C_4H_9$ | S | H | H | 76,284 |
| 5 | " | " | " | n-$C_4H_9$ | S | H | H | 75,873 |
| 6 | " | i-$C_3H_7$ | " | i-$C_4H_9$ | S | H | H | 74,943 |
| 7 | " | $C_2H_5$ | " | sec.-$C_4H_9$ | S | H | H | 76,264 |
| 8 | " | i-$C_3H_7$ | " | " | S | H | H | 74,929 |
| 9 | " | $C_2H_5$ | n-$C_3H_7$ | " | S | H | H | 76,364 |
| 10 | " | i-$C_3C_7$ | " | " | S | H | H | 74,947 |
| 11 | " | $C_2H_5$ | n-$C_4H_9$ | i-$C_4H_9$ | S | H | H | 76,378 |
| 12 | " | i-$C_3H_7$ | " | " | S | H | H | 74,953 |
| 13 | " | $C_2H_5$ | i-$C_4H_9$ | " | S | H | H | 76,879 |
| 14 | " | i-$C_3H_7$ | " | " | S | H | H | 75,426 |
| 15 | " | $C_2H_5$ | sec.-$C_4H_9$ | " | S | H | H | 75,895 |
| 16 | " | i-$C_3H_7$ | " | " | S | H | H | 74,569 |
| 17 | " | $C_2H_5$ | $CH_2CH_2OCH_3$ | " | S | H | H | 76,420 |
| 18 | " | i-$C_3H_7$ | " | " | S | H | H | 74,962 |
| 19 | " | " | n-$C_3H_7$ | n-$C_4H_9$ | S | H | H | |
| 20 | " | $C_2H_5$ | " | i-$C_4H_9$ | S | 4-F | H | |
| 21 | " | i-$C_3H_7$ | " | " | S | 4-F | H | |
| 22 | " | $C_2H_5$ | " | " | S | 3-Cl | H | 76,231 |
| 23 | " | i-$C_3H_7$ | " | " | S | 3-Cl | H | 74,828 |
| 24 | " | $C_2H_5$ | " | tert-$C_4H_9$ | S | H | H | 76,021 |

Tabelle 1 (fortgesetzt)

| Bsp.Nr. | R | R¹ | R² | R³ | X | Y | Z | $^{31}$P-NMR Verschiebung (ppm) bzw. Brechungs-index $n_D^{20}$ |
|---|---|---|---|---|---|---|---|---|
| 25 | " | i-$C_3H_7$ | " | " | S | H | H | 74,517 |
| 26 | " | $C_2H_5$ | i-$C_3H_7$ | i-$C_4H_9$ | S | H | H | |
| 27 | " | i-$C_3H_7$ | " | " | S | H | H | |
| 28 | $C_2H_5$ | i-$C_3H_7$ | $CH_2CH{=}CH_2$ | i-$C_4H_9$ | S | H | H | 74,604 |
| 29 | " | $C_2H_5$ | " | " | S | H | H | 75,870 |
| 30 | " | i-$C_3H_7$ | n-$C_3H_7$ | n-$C_4H_9$ | S | H | H | |
| 31 | $C_2H_5$ | $C_2H_5$ | n-$C_3H_7$ | i-$C_4H_9$ | S | 3F | H | $n_D^{20}$ = 1,5064 |
| 32 | $C_2H_5$ | i-$C_3H_7$ | n-$C_3H_7$ | i-$C_4H_9$ | S | 3F | H | $n_D^{20}$ = 1,5003 |
| 33 | $C_2H_5$ | $C_2H_5$ | n-$C_3H_7$ | i-$C_4H_9$ | S | 3Cl | 4Cl | $n_D^{20}$ = 1,5274 |
| 34 | $C_2H_5$ | i-$C_3H_7$ | n-$C_3H_7$ | i-$C_4H_9$ | S | 3Cl | 4Cl | $n_D^{20}$ = 1,5239 |

Die Herstellung der als Vorprodukte einzusetzenden neuen N-Alkyl-glycinanilide der Formel (IV) soll wie folgt erläutert werden:

**Beispiel a:**

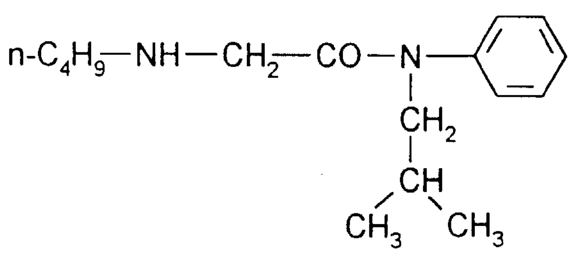

Zu 30 ml n-Butylamin gibt man 11,3 g (0,05 Mol) Chloressigsäure-N-isobutylanilid (Herstellung s. Pestic. Chem., Proc. Int. Congr. Pestic. Chem., 2nd 1972, 5, 177-188; CA 80 (1974) 67293 t) und läßt die Mischung 18 Stunden bei Raumtemperatur (ca. 20°C) stehen. Das überschüssige Butylamin wird im Vakuum abdestilliert, den Rückstand löst man in 100 ml Methylenchlorid und extrahiert zweimal mit je 50 ml Wasser. Die organische Phase wird über Natriumsulfat getrocknet und im Vakuum eingedampft. Man erhält 10,5 g (80 % der Theorie) N-n-Butylamino-essigsäure-N-isobutylanilid als farbloses Öl mit dem Retentionsindex (OV) 1861.

Gemäß Beispiel a werden die folgenden Verbindungen der Formel (IV)

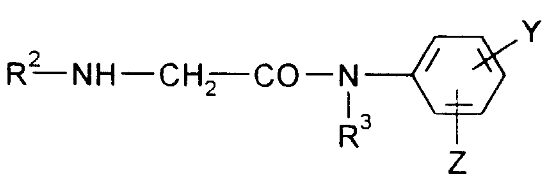

(IV)

erhalten (Tabelle 2):

Tabelle 2

| Beispiel | $R^2$ | $R^3$ | Y | Z | GC-Index (OV) bzw. Brechungsindex $n_D^{20}$ |
|---|---|---|---|---|---|
| b | n-$C_3H_7$ | iso-$C_4H_9$ | H | H | 1824 |
| c | n-$C_3H_7$ | n-$C_4H_9$ | H | H | 1872 |
| d | $C_2H_5$ | iso-$C_4H_9$ | H | H | 1734 |
| e | $C_2H_5$ | n-$C_4H_9$ | H | H | 1758 |
| f | $C_2H_5$ | sec-$C_4H_9$ | H | H | 1745 |
| g | n-$C_3H_7$ | sec-$C_4H_9$ | H | H | 1832 |
| h | iso-$C_4H_9$ | iso-$C_4H_9$ | H | H | 1868 |
| i | sec-$C_4H_9$ | iso-$C_4H_9$ | H | H | 1852 |
| j | $-CH_2-CH_2-OCH_3$ | iso-$C_4H_9$ | H | H | 1845 |
| k | $-CH-CH=CH_2$ | iso-$C_4H_9$ | H | H | 1810 |
| l | n-$C_3H_7$ | t-$C_4H_9$ | H | H | 1749 |
| m | n-$C_3H_7$ | iso-$C_4H_9$ | 3-Cl | H | |
| n | n-$C_3H_7$ | iso-$C_4H_9$ | 4-F | H | |
| o | n-$C_3H_7$ | iso-$C_4H_9$ | 3F | H | $n_D^{20}$ = 1,5002 |
| p | n-$C_3H_7$ | iso-$C_4H_9$ | 3Cl | 4Cl | $n_D^{20}$ = 1,5316 |

Die biologische Wirkung der erfindungsgemäßen Verbindungen der Formel (I) sol anhand der folgenden Beispiele erläutert werden:

**Beispiel A**

| Grenzkonzentrationstest / Bodeninsekten | |
|---|---|
| Testinsekt | Diabrotica balteata - Larven im Boden |
| Lösungsmittel | 4 Gewichtsteile Aceton |
| Emulgator | 1 Gewichtsteil Alkylarylpolyglykolether |

Zur Herstellung einer zweckmäßigen Wirkstoffzubereitung vermischt man 1 Gew.-Teil Wirkstoff mit der angegebenen Menge Lösungsmittel, gibt die angegebene Menge Emulgator zu und verdünnt das Konzentrat mit Wasser auf die gewünschte Konzentration. Dabei spielt die Konzentration des Wirkstoffes in der Zubereitung praktisch keine Rolle, entscheidend ist allein der Wirkstoffgewichtsmenge pro Volumeneinheit Boden, welche in ppm (mg/l) angegeben wird. Man füllt den Boden in 0,5 1 Töpfe und läßt diese bei 20°C stehen.

Sofort nach dem Ansatz werden je Topf 5 vorgekeimte Maiskörner ausgelegt. Nach 1 Tag werden in den behandelten Boden die entsprechenden Testinsekten gesetzt. Nach weiteren 7 Tagen wird der Wirkungsgrad des Wirkstoffes durch Auszählen der toten und lebenden Testinsekten in % bestimmt. Der Wirkungsgrad ist 100 %, wenn alle Testinsekten abgetötet sind, er ist 0 %, wenn noch genau so viele Testinsekten leben wie bei der unbehandelten Kontrolle.

In diesem Test zeigten beispielsweise die Verbindungen der Herstellungsbeispiele 2, 6 und 8 bei einer beispielhaften Wirkstoffkonzentration von 20 ppm einen Wirkungsgrad von 100 %.

**Beispiel B**

| Grenzkonzentrationstest / Nematoden | |
|---|---|
| Testnematode | Meloidogyne incognita |
| Lösungsmittel | 4 Gewichtsteile Aceton |
| Emulgator | 1 Gewichtsteil Alkylarylpolyglykolether |

Zur Herstellung einer zweckmäßigen Wirkstoffzubereitung vermischt man 1 Gew.-Teil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt, der mit den Testnematoden stark verseucht ist. Dabei spielt die Konzentration des Wirkstoffes in der Zubereitung praktisch keine Rolle, entscheidend ist allein die Wirkstoffmenge pro Volumeneinheit Boden, welche in ppm (= mg/l) angegeben wird. Man füllt den behandelten Boden in Töpfe, sät Salat ein und hält die Töpfe bei einer Gewächshaustemperatur von 25°C.

Nach sechs Wochen werden die Salatwurzeln auf Nematodenbefall (Wurzelgallen) untersucht und der Wirkungsgrad des Wirkstoffes in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

In diesem Test zeigten die erfindungsgemäßen Verbindungen eine gute Wirksamkeit.

**Beispiel C**

| Myzus-Test | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gew.-Teil Wirkstoff mit der angegebenen Menge Lösungsmittel, gibt die angegebene Menge Emulgator zu und verdünnt das Konzentrat mit Wasser auf die gewünschte Konzentration.

Kohlblätter (Brassica oleracea), die stark von der Pfirsischblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

In diesem Test zeigte z.B. die Verbindung des Herstellungsbeispiels 29 bei einer beispielhaften Wirkstoffkonzentration von 0,01 % nach einem Tag eine Wirkung von 100 %.

**Beispiel D**

| Nephotettix-Test | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryzae sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit bestimmt man die Abtötung in %. Dabei bedeutet 100%, daß alle Zikaden abgetötet wurden; 0 % bedeutet, daß keine Zikaden abgetötet wurden.

Bei diesem Test zeigten z.B. die Verbindungen gemäß den Herstellungsbeispielen 4, 5, 6, 7, 9, 11, 14, 16, 17, 18, 28 und 29 bei einer beispielhaften Wirkstoffkonzentration von 0,2% nach 6 Tagen einen Abtötungsgrad von 100 %.

**Beispiel E**

| Tetranychus-Test (OP-resistent) | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe (Tetranychus urticae) befallen sind, werden in eine Wirkstoffzubereitung der gewünschten Konzentration getaucht.

Nach der gewünschten Zeit wird die Abtötung in % bestimmt. Dabei bedeutet 100%, daß alle Spinnmilben abgetötet wurden; 0 % bedeutet, daß keine Spinnmilben abgetötet wurden.

Bei diesem Test zeigten z.B. die Verbindungen der Herstellungsbeispiele 2, 4, 6, 7, 8, 9, 13, 14, 18, 26, 28 und 29 bei einer beispielhaften Wirkstoffkonzentration von 0,01% nach 7 Tagen eine Wirkung von 100 %.

**Beispiel F**

| Test mit Fliegen (Musca domestica) | |
|---|---|
| Testinsekten | adulte Musca domestica, Stamm WHO (N) |
| Emulgator | 35 Gewichtsteile Ethylenglykolmonomethylether |
| | 35 Gewichtsteile Nonylphenolpolyglykolether |

Zur Herstellung einer geeigneten Formulierung vermischt man drei Gewichtsteile Wirkstoff mit siebenTeilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierschalen (Ø 9,5 cm) pipettiert, die sich in Petrischalen entsprechender Größe befinden. Nach Trocknung der Filterscheiben werden 25 Testinsekten in die Petrischalen überführt und abgedeckt.

Nach 1, 3, 5 und 24 Stunden wird die Wirksamkeit der Wirkstoffzubereitung ermittelt. Dabei bedeutet 100 %, daß alle Fliegen abgetötet wurden; 0 % bedeutet, daß keine Fliegen abgetötet wurden.

Bei diesem Test zeigte z.B. die Verbindung des Herstellungsbeispieles 11 bei einer beispielhaften Wirkstoffkonzentration von 1000 ppm eine 100%ige Wirkung.

**Beispiel G**

| Test mit Boophilus microplus resistent/SP-resistenter Parkhurst-Stamm | |
|---|---|
| Testinsekten | adulte gesogene Weibchen |
| Lösungsmittel | 35 Gewichtsteile Ethylenglykolmonomethylether 35 Gewichtsteile Nonylphenolpolyglykolether |

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

10 adulte Boophilus microplus res. werden in die zu testende Wirkstoffzubereitung 1 Minute getaucht. Nach

Überführung in Plastikbecher und Aufbewahrung in einem klimatisierten Raum wird der Abtötungsgrad bestimmt.

Dabei bedeutet 100 %, daß alle Zecken abgetötet wurden; 0 % bedeutet, daß keine Zecken abgetötet wurden.

Bei diesem Test zeigten z.B. die Verbindungen der Herstellungsbeispiele 9 und 10 bei einer beispielhaften Wirkstoffkonzentration von 1000 ppm eine 100%ige Wirkung.

## Patentansprüche

**1.** Phosphorsäure-Derivate der allgemeinen Formel (I)

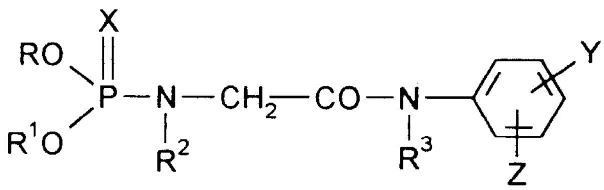

(I)

in welcher

R für gegebenenfalls substituiertes Alkyl steht,

$R^1$ für gegebenenfalls substituiertesAlkyl steht,

$R^2$ für gegebenenfalls substituiertes Alkyl oder gegebenenfalls substituiertes Alkenyl steht,

$R^3$ für gegebenenfalls substituiertes $C_4$-$C_6$-Alkyl steht,

X für Sauerstoff oder Schwefel steht, und

Y und Z für gleiche oder verschiedene Reste aus der Reihe Wasserstoff, Halogen, Alkyl und Alkoxy stehen.

**2.** Phosphorsäure-Derivate gemäß Anspruch 1, wobei in Formel (I)

R für $C_1$-$C_6$-Alkyl, welches gegebenenfalls durch Halogen und/oder $C_1$-$C_4$-Alkoxy substituiert ist steht,

$R^1$ für $C_1$-$C_6$-Alkyl, welches gegebenenfalls durch Halogen und/oder $C_1$-$C_4$-Alkoxy substituiert ist, steht,

$R^2$ für $C_1$-$C_6$-Alkyl oder $C_2$-$C_6$-Alkenyl, wobei diese Reste gegebenenfalls durch Halogen und/oder $C_1$-$C_4$-Alkoxy substituiert sind, steht,

$R^3$ für $C_4$-$C_6$-Alkyl, welches gegebenenfalls durch Halogen und/oder $C_1$-$C_4$-Alkoxy substituiert ist, steht und

Y und Z für Wasserstoff, Halogen oder für gleiche oder verschiedene Reste aus der Reihe $C_1$-$C_6$-Alkyl und $C_1$-$C_6$-Alkoxy stehen.

**3.** Phosphorsäure-Derivate gemäß Anspruch 1, wobei in Formel (I)

R für $C_1$-$C_4$-Alkyl steht,

$R^1$ für $C_1$-$C_4$-Alkyl steht,

$R^2$ für $C_1$-$C_4$-Alkyl, $C_1$-$C_2$-Alkoxy-$C_1$-$C_4$-Alkyl oder Allyl steht,

$R^3$ für $C_4$-Alkyl steht und

Y und Z für Wasserstoff, stehen oder

Y für Wasserstoff steht und Z für Fluor oder Chlor steht.

**4.** Phosphorsäure-Derivate gemäß Anspruch 1, wobei in Formel (I)

x für Schwefel steht.

**5.** Verfahren zur Herstellung der Phosphorsäure-Derivate der allgemeinen Formel (I) gemäß Anspruch 1, dadurch gekennzeichnet, daß man

a) Verbindungen der allgemeinen Formel (II)

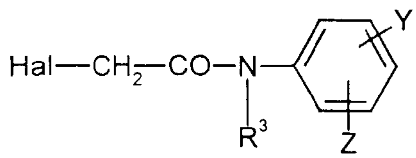

(II),

in welcher

$R^3$, Y und Z die in Anspruch 1 angegebenen Bedeutungen haben, und
Hal für Halogen steht,

mit der Verbindung der allgemeinen Formel (III)

$$R^2\text{-}NH_2 \qquad \text{(III)}$$

in welcher

$R^2$ die in Anspruch 1 angegebene Bedeutung hat,

umsetzt, und

b) die im Reaktionsschritt a) erhaltenen Verbindungen der allgemeinen Formel (IV)

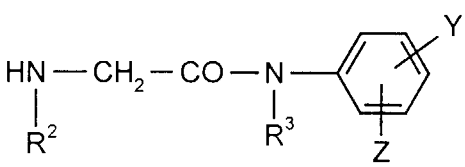

(IV)

in welcher

$R^2$, $R^3$, Y und Z die in Anspruch 1 angegebenen Bedeutungen haben,

gegebenenfalls nach ihrer Isolierung, mit Verbindungen der allgemeinen Formel (V)

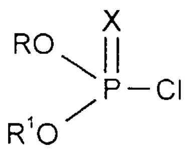

(V)

in welcher

$R$, $R^1$ und X die in Anspruch 1 angegebenen Bedeutungen haben,

gegebenenfalls in Gegenwart eines Säureakzeptors und gegebenenfalls in Gegenwart eines Verdünnungsmittels umsetzt.

**6.** Schädlingsbekämpfungsmittel, gekennzeichnet durch einen Gehalt an mindestens einer Verbindung der Formel (I) gemäß Anspruch 1.

**7.** Verwendung von Verbindungen der Formel (I) gemäß Anspruch 1 zur Bekämpfung von Schädlingen.

**8.** Verfahren zur Bekämpfung von Schädlingen, dadurch gekennzeichnet, daß man Verbindungen der Formel (I) gemäß den Anspruch 1 auf die Schädlinge und/oder ihren Lebensraum einwirken läßt.

**9.** Verfahren zur Herstellung von Schädlingsbekämpfungsmitteln, dadurch gekennzeichnet, daß man Verbindungen der Formel (1) gemäß Anspruch 1 mit Streckmitteln und/oder oberflächenaktiven Mitteln vermischt.

**10.** Verbindungen der allgemeinen Formel (IV)

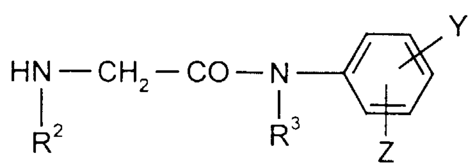

(IV)

in welcher

$R^2$, $R^3$, Y und Z die in Anspruch 1 angegebenen Bedeutungen haben.

## Claims

**1.** Phosphoric acid derivatives of the general formula (I)

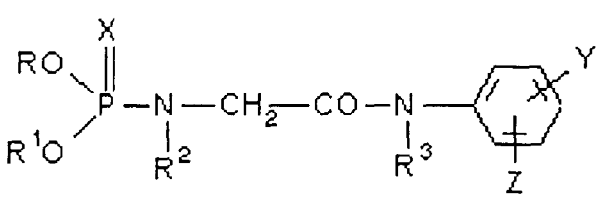

(I)

in which

R represents optionally substituted alkyl;

| | |
|---|---|
| $R^1$ | represents optionally substituted alkyl; |
| $R^2$ | represents optionally substituted alkyl or optionally substituted alkenyl; |
| $R^3$ | represents optionally substituted $C_4$-$C_6$-alkyl; |
| X | represents oxygen or sulphur, and |
| Y and Z | represent identical or different radicals from the series comprising hydrogen, halogen, alkyl and alkoxy. |

**2.** Phosphoric acid derivatives according to Claim 1, in which, in formula (I),

| | |
|---|---|
| R | represents $C_1$-$C_6$-alkyl which is optionally substituted by halogen and/or $C_1$-$C_4$-alkoxy, |
| $R^1$ | represents $C_1$-$C_6$-alkyl which is optionally substituted by halogen and/or $C_1$-$C_4$-alkoxy, |
| $R^2$ | represents $C_1$-$C_6$-alkyl or $C_2$-$C_6$-alkenyl, these radicals optionally being substituted by halogen and/or $C_1$-$C_4$-alkoxy, |
| $R^3$ | represents $C_4$-$C_6$-alkyl which is optionally substituted by halogen and/or $C_1$-$C_4$-alkoxy, and |
| Y and Z | represent hydrogen, halogen or identical or different radicals from the series comprising $C_1$-$C_6$-alkyl and $C_1$-$C_6$-alkoxy. |

**3.** Phosphoric acid derivatives according to Claim 1, in which, in formula (I),

| | |
|---|---|
| R | represents $C_1$-$C_4$-alkyl, |
| $R^1$ | represents $C_1$-$C_4$-alkyl, |
| $R^2$ | represents $C_1$-$C_4$-alkyl, $C_1$-$C_2$-alkoxy-$C_1$-$C_4$-alkyl or allyl, |
| $R^3$ | represents $C_4$-alkyl, and |
| Y and Z | represent hydrogen or |
| Y | represents hydrogen and Z represents fluorine or chlorine. |

**4.** Phosphoric acid derivatives according to Claim 1, in which, in formula (I),

X represents sulphur.

**5.** Process for the preparation of the phosphoric acid derivatives of the general formula (I) according to Claim 1, characterized in that

a) compounds of the general formula (II)

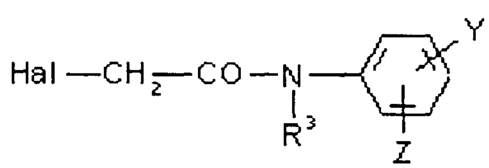

(II),

in which

$R^3$, Y and Z have the meanings given in Claim 1 and

Hal represents halogen,

are reacted with the compound of the general formula (III)

$$R^2\text{-}NH_2 \quad (III)$$

in which

$R^2$ has the meaning given in Claim 1,

and

b) the compounds of the general formula (IV)

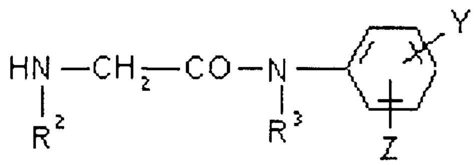

(IV)

in which

$R^2$, $R^3$, Y and Z have the meanings given in Claim 1,

which have been obtained in reaction step a),
are reacted, if appropriate after their isolation, with compounds of the general formula (V)

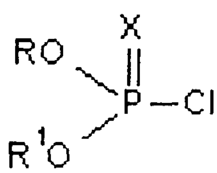

(V)

in which

R, $R^1$ and X have the meanings given in Claim 1,

if appropriate in the presence of an acid acceptor and if appropriate in the presence of a diluent.

**6.** Pesticides, characterized in that they contain at least one compound of the formula (I) according to Claim 1.

**7.** Use of compounds of the formula (I) according to Claim 1 for combating pests.

**8.** Method of combating pests, characterized in that compounds of the formula (I) according to Claim 1 are allowed to act on the pests and/or their environment.

**9.** Process for the preparation of pesticides, characterized in that compounds of the formula (I) according to Claim 1 are mixed with extenders and/or surface-active agents.

**10.** Compounds of the general formula (IV)

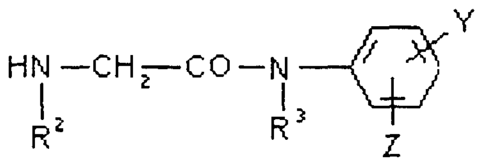

(IV)

in which

$R^2$, $R^3$, Y and Z have the meanings given in Claim 1.

## Revendications

**1.** Dérivés d'acide phosphorique de formule générale (I)

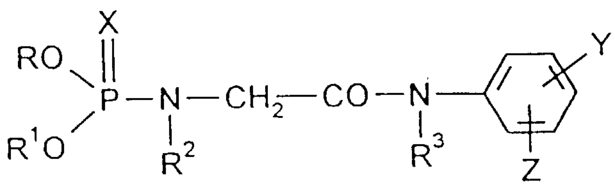

(I)

dans laquelle

| | |
|---|---|
| R | est un groupe alkyle éventuellement substitué, |
| $R^1$ | est un groupe alkyle éventuellement substitué, |
| $R^2$ | est un groupe alkyle éventuellement substitué ou un groupe alcényle éventuellement substitué, |
| $R^3$ | est un groupe alkyle en $C_4$ à $C_6$ éventuellement substitué, |
| x | est l'oxygène ou le soufre et |
| Y et Z | représentent des restes identiques ou différents de la série hydrogène, halogène, alkyle et alkoxy. |

**2.** Dérivés d'acide phosphorique suivant la revendication 1, dans la formule (I) desquels

| | |
|---|---|
| R | est un reste alkyle en $C_1$ à $C_6$ qui est substitué le cas échéant par un halogène et/ou un radical alkoxy en $C_1$ à $C_4$, |
| $R^1$ | est un reste alkyle en $C_1$ à $C_6$ qui est substitué le cas échéant par un halogène et/ou un radical alkoxy en $C_1$ à $C_4$, |
| $R^2$ | est un reste alkyle en $C_1$ à $C_6$ ou un reste alcényle en $C_2$ à $C_6$, ces restes étant substitués le cas échéant par un halogène et/ou un radical alkoxy en $C_1$ à $C_4$, |
| $R^3$ | est un reste alkyle en $C_4$ à $C_6$ qui est substitué le cas échéant par un halogène et/ou un radical alkoxy en $C_1$ à $C_4$ et |
| Y et Z | représentent l'hydrogène, un halogène ou des restes identiques ou différents de la série alkyle en $C_1$ à $C_6$, et alkoxy en $C_1$ à $C_6$. |

**3.** Dérivés d'acide phosphorique suivant la revendication 1, dans la formule (I) desquels

| | |
|---|---|
| R | est un reste alkyle en $C_1$ à $C_4$, |
| $R^1$ | est un reste alkyle en $C_1$ à $C_4$, |
| $R^2$ | est un reste alkyle en $C_1$ à $C_4$ (alkoxy en $C_1$ ou $C_2$)-(alkyle en $C_1$ à $C_4$) ou allyle, |
| $R^3$ | est un reste alkyle en $C_4$ et |
| Y et Z | représentent l'hydrogène, ou bien |
| Y | est l'hydrogène et Z est le fluor ou le chlore. |

4. Dérivés d'acide phosphorique suivant la revendication 1, dans la formule (I) desquels

X est le soufre.

5. Procédé de production des dérivés d'acide phosphorique de formule générale (I) suivant la revendication 1, caractérisé en ce que

a) on fait réagir les composés de formule générale (II)

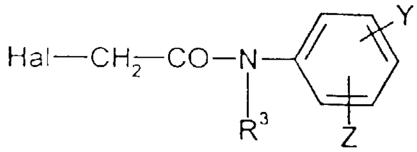

(II),

dans laquelle

$R^3$, Y et Z ont les définitions indiquées dans la revendication 1 et
Hal représente un halogène,

avec le composé de formule générale (III)

$$R^2\text{-}NH_2 \quad \text{(III)}$$

dans laquelle

$R^2$ a la définition indiquée dans la revendication 1,

et
b) on fait réagir les composés obtenus dans l'étape réactionnelle a), de formule générale (IV)

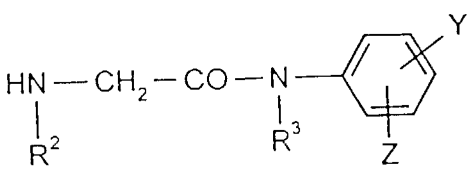

(IV)

dans laquelle
$R^2$, $R^3$, Y et Z ont les définitions indiquées dans la revendication 1,
le cas échéant après les avoir isolés, avec des composés de formule générale (V)

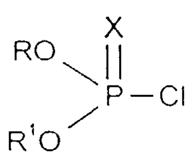

(V)

dans laquelle
R, $R^1$ et X ont les définitions indiquées dans la revendication 1,
le cas échéant en présence d'un accepteur d'acide et en la présence éventuelle d'un diluant.

**6.** Compositions pesticides, caractérisées par une teneur en au moins un composé de formule (I) suivant la revendication 1.

**7.** Utilisation de composés de formule (I) suivant la revendication 1 pour combattre des parasites.

**8.** Procédé pour combattre des parasites, caractérisé en ce qu'on fait agir des composés de formule (I) suivant la revendication 1 sur les parasites et/ou sur leur milieu.

**9.** Procédé de préparation de compositions pesticides, caractérisé en ce qu'on mélange des composés de formule (I) suivant la revendication 1 avec des diluants et/ou des agents tensio-actifs.

**10.** Composés de formule générale (IV)

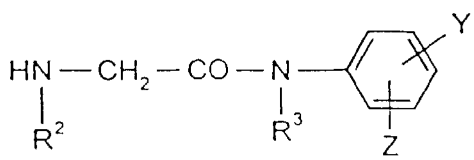

(IV)

dans laquelle

$R^2$, $R^3$, Y et Z ont les définitions indiquées dans la revendication 1.